# EUROPEAN PATENT APPLICATION

(11) **EP 2 028 267 A1**
(43) Date of publication of application: **25.02.2009**
(21) Application number: 07016358.9
(22) Date of filing: 21.08.2007
(51) Int. Cl.: C12N 5/06, A61K 35/14

(54) **Method of producing homologous cellular blood components from hematopoietic progenitor cells**

(71) Applicant: Charité-Universitätsmedizin Berlin, 10117 Berlin (DE)
(72) Inventor: Moldenhauer, Anja, 10117 Berlin (DE); Salama, Abdulgabar, 10117 Berlin (DE); Kiesewetter, Holger, 10117 Berlin (DE)
(74) Representative: Engelhard, Markus

(57) **Abstract**

The present invention relates to an autologous cell culture system for the production of homologous cellular blood components, namely granulocytes, thrombocytes, and erythrocytes, using endothelial cells (EC) from the umbilical cord and hematopoietic progenitor cells (HPC) from the umbilical cord blood of a single donor.

## Description

The present invention relates to an autologous cell culture system for the production of homologous cellular blood components, namely granulocytes, thrombocytes, and erythrocytes, using endothelial cells (EC) from the umbilical cord and hematopoietic progenitor cells (HPC) from the umbilical cord blood of a single donor.

Each year 4.45 million erythrocyte concentrates, 381.000 thrombocyte concentrates, and about 500-1000 granulocyte concentrates are transfused in Germany. A decrease in the willingness to donate blood as well as patients with rare blood types represent major challenges in modem transfusion medicine. Especially erythrocyte concentrates of the blood type 0 (Rh-), which can be transfused without prior cross-matching in an emergency, is of short supply. Moreover, an increasing number of mandatory tests of blood products for infectious diseases as well as procedures for the inactivation of pathogens increase the cost per transfusion unit. Despite all safety measures there still remains a risk of transmission of rare diseases or of diseases for which no inexpensive screening tests exist (e.g. West Nile Virus, Chikungunya, SARS, Avian influenza, BSE, etc.).
Other blood products, such as thrombocyte and granulocyte concentrates, require expensive apheresis systems. The production of granulocyte concentrates comprises the interventional sedimentation of erythrocytes using hydroxyethyl starch (HES) (Price T.H. et al., Blood 95 (2000) 3302-3309; Stroncek D.F. et al., Transfusion 41 (2001) 1037-1044). Due to the side effects of HES, which can range from allergic reactions to liquid retention and peripheral edemas, a maximum of only four granulocyte aphereses is allowed per donor per year in Germany (Bundesärztekammer, W.B.d., Deutsches Ärzteblatt 94 (1999) 1268-1276). Therefore, alternative sources for cellular blood components are of great interest.

Interleukins are proinflammatory cytokines, which are synthesized by endothelial cells, stroma cells and macrophages. Interleukin-1 is known to stimulate endothelial secretion of granulocyte and granulocyte-macrophage colony stimulating factor (Broudy V.C. et al., J Immunol 139 (1987) 464-468). Interleukin-3 and-6 are also known to induce the expansion of hematopoietic progenitor cells (Ikebuchi K. et al., Proc Natl Acad Sci USA 84 (1987) 9035-9039; Rossmanith T. et al., Stem Cells 19 (2001) 313-320), and Interleukin-11 seems to be relevant for the expansion and differentiation of thrombocytes (De Bruyn C. et al., Stem Cells Dev 14 (2005) 415-424). Insulin-like growth factor (IGF) 1 and 2 are polypeptide hormones similarly structured as insulin and they both regulate cell growth in similar ways as insulin (Zapf J. et al., Clin Endocrinol Metab 13 (1984) 3-30). They play an important role in bone formation by activating osteocytes (Rodriguez S. et al., Hum Genet 122 (2007) 1-21). IGF-1 is synthesized by liver cells affecting almost every tissue in the body (lung, muscle, cartilage, bone, liver, kidney, nerves) (Underwood L.E. and D'Ercole A.J., Clin Endocrinol Metab 13 (1984) 69-89). Stroma-derived factor 1, also called CXCL 12, is a small chemokine secreted by stroma and endothelial cells (Lapidot T. and Kollet O., Leukemia 16 (2002) 1992-2003). Both isoforms, SDF-1α and β result from different splicing of the same gene (Shirozu M. et al., Genomics 28 (1995) 494-500) and play an important role in the homing of hematopoietic progenitor cells to the bone marrow after mobilization (Juarez J. and Bendall L., Histol Histopathol 19 (2004) 299-309). They are also responsible for the migration of hematopoietic progenitor cells from the fetal liver to the bone marrow. Erythropoietin and thrombopoietin are glycoportein hormones responsible for the differentiation of erythrocytes and thrombocytes from precursor cells, respectively. Both are produced in liver and kidney. Erythropoietin is clinically used in patients with chronic anemia due to renal failure or cancer chemotherapy (Goodnough L.T., Exp Hematol 35 (2007) 167-172). Its direct application has recently been under governmental scrutiny in the US (Fishbane S. and Nissenson A.R., Kidney Int. (2007)). Thrombopoietin, formerly called megakaryocyte growth factor, induces the proliferation of hematopoietic stem cells. It is responsible for platelet progenitor differentiation (megakaryocyte) (Kaushansky K., N Engl J Med 339 (1998) 746-754). In several trials, its high immunogenicity became apparent which prevents its clinical administration (Kuter D.J., Blood 109 (2007) 4607-4616). Like erythropoietin and thrombopoitein, granulocyte-colony stimulating-factor (G-CSF) can be applied in order to achieve a fast neutrophil recovery (Marsh J.C. et al., Semin Hematol 44 (2007) 138-147), hematopoietic progenitor cell and granulocyte mobilization (Price T.H. et al., Blood 95 (2000) 3302-3309; Petit I., Nat Immunol 3 (2002) 687-694).

The differentiation of blood hematopoietic progenitor cells into mature erythrocytes, granulocytes, or thrombocytes is not new. Previous differentiation approaches comprise the cultivation of said progenitor cells in cytokine cocktails. The granulocyte colony stimulating factor and the granulocyte macrophage stimulating factor allow the differentiation of granulocytes (Souza L.M. et al., Science 232 (1986) 61-65). The addition of erythropoietin, interleukin-3 and -6 as well as stem cell factor in combination with insulin-like growth factor 1 promotes the differentiation of erythrocytes (Cheung J.O. et al., J Immunol Methods 319 (2007) 104-117). The use of thrombopoietin in combination with Flt-3 ligand, interleukin-6, - 9 and/or -11 results in the differentiation of thrombocytes (Cortin V. et al., Exp Hematol 33 (2005) 1182-1191; De Bruyn C. et al., Stem Cells Dev 14 (2005) 415-424). Common to all of these techniques is the use of highly concentrated cytokine combinations, which have to be produced in accordance with the guidelines of clinical practice, since they are destined for a clinical application. Therefore, the production of cellular blood components from blood progenitor cells is very expensive, laborious and resource-consuming.

Another approach includes the combination (= co-culture) of blood progenitor cells and human bone marrow stroma cell lines (Takagi M. J Biosci Bioeng 99 (2005) 189-196). However, this system either uses murine stroma cells (Takagi M. J Biosci Bioeng 99 (2005) 189-196) or the application of additional cytokine combinations (Gammaitoni L. et al., Exp Hematol 31 (2003) 261-270). Both are unsuitable for clinical applications, since stem and stroma cells are usually from different species or individuals (= donors).

Accordingly, it was an object of the present invention to provide for an autologous cell culture system for the production of homologous cellular blood components (granulocytes, thrombocytes, erythrocytes), which can be established without any severe invasive procedures. It was also an object of the present invention to provide for a process of producing cellular blood components that is easy to perform and that achieves good yields of said cellular blood components.

The objects of the present invention are solved by a method of producing homologous cellular blood components from hematopoietic progenitor cells (HPC), said method comprising
collecting vein endothelial cells (EC) from the umbilical cord and hematopoietic progenitor cells from the umbilical cord blood of a single individual,
culturing said vein endothelial cells in the presence of a single stimulant,
collecting the supernatant of said culture comprising said vein endothelial cells and said single stimulant,
culturing said hematopoietic progenitor cells in the presence of said supernatant, and
collecting said cellular blood component from said culture comprising said hematopoietic progenitor cells and said supernatant.

In one embodiment of the present invention said single individual is human.

In one embodiment said hematopoietic progenitor cells are CD34(+) or CD133(+) cells.

In one embodiment said cellular blood component is selected from the group comprising granulocytes, thrombocytes, and erythrocytes.

In one embodiment said cellular blood component are granulocytes, wherein, preferably, said single stimulant is interleukin-1 or interleukin-3.

In one embodiment said cellular blood component are thrombocytes, wherein, preferably, said single stimulant is selected from the group comprising stroma-derived factor 1, thrombopoietin, fibroblastic growth factor 4, and interleukin-11.

In one embodiment said cellular blood component are erythrocytes, wherein, preferably, said single stimulant is selected from the group comprising insulin-like growth factor 1, insulin-like growth factor 2, and erythropoietin.

In one embodiment said vein endothelial cells are cultured in the presence of a single stimulant for 15 to 24 hours.

In one embodiment said hematopoietic progenitor cells are cultured in the presence of said supernatant for ≤ 14 days.

In one embodiment of the present invention said hematopoietic progenitor cells are cultured in the presence of said supernatant and further matured after expansion by addition of a single maturation stimulant, wherein preferably said single maturation stimulant is G-CSF for granulocytes, thrombopoietin for thrombocytes, and erythropoietin for erythrocytes.

In one embodiment the yield of said cellular blood component in relation to the initial number of said hematopoietic progenitor cells, is:
a) in the case of granulocytes, more than 100-fold, preferably more than 500-fold, even more preferably more than 1000-fold,
b) in the case of thrombocytes, more than 1000-fold, preferably more than 5000-fold, even more preferably more than 10000-fold, and
c) in the case of erythrocytes, more than 10000-fold, preferably more than 50000-fold, even more preferably more than 100000-fold.

The objects of the present invention are also solved by a homologous cellular blood component, selected from the group comprising granulocytes, thrombocytes and erythrocytes and produced by the method according to the present invention.

The objects of the present invention are also solved by the use of said homologous cellular blood component for transfusion.

The term "cellular blood component", as used herein, is meant to refer to different types of cells in the blood, which exhibit very different functions, ranging from the transport of oxygen (erythrocytes) to the initiation of blood clotting (thrombocytes) and immune defense (granulocytes).

"Homologous cellular blood component" is meant to refer to blood cells of the same blood type.

The term "hematopoietic progenitor cells", as used herein, is meant to refer to multipotent cells involved in the process of hematopoiesis, which, unlike pluripotent hematopoietic stem cells, are limited in the number of times that they can divide before differentiating to form mature blood cells.

The term "autologous culture system", as used herein, is meant to refer to a system for producing and culturing cellular blood components of an individual from his/her own body. The term "stimulant" or "maturation stimulant" is meant to refer to factors/substances that support hematopoiesis and/or promote the differentiation into mature blood cells.

The term "a single stimulant" is meant to refer to a stimulant which, at the time of its administration and presence in a culture, is not used in combination with other such stimulants.

The term "the yield of said cellular blood component", as used herein, is meant to refer to the total output of cells or the total number of cells of the mature cellular blood component in a given cell culture.

The term "single individual" is meant to refer to a scenario wherein the endothelial cells and the hematopoietic progenitor cells are obtained from the same individual.

The inventors have surprisingly found that culturing hematopoietic progenitor cells in supernatants of specifically stimulated endothelial cells, wherein both cell types are collected from the umbilical cord and cord blood of a single, i.e. the same individual, provides an easy, efficient, and inexpensive way of producing homologous cellular blood components, namely granulocytes, thrombocytes, and erythrocytes.
Cellular blood components obtained by the method according to the present invention are optimally suited for clinical applications, since both HPC and EC are derived from a single donor. Furthermore, an autologous culture system using umbilical cord blood HPC and umbilical cord EC according to the present invention can be established without any severe invasive measures, such as the use of HES, previously used for the collection of granulocytes from healthy donors.
In addition, the yield of said cellular blood component obtained by the method according to the present invention is equivalent or substantially higher than those obtained by presently used procedures. Another advantage of the present invention is the independence from blood donor availability.
Due to the reduction of the amount of cytokines that need to be applied and the decrease in the number of diagnostic tests that have to be performed, the cost efficiency of the production of cellular blood components according to the present invention is significantly increased.

Another advantage of the present invention is the fact that said supernatant of specifically stimulated EC can be easily stored at 4°C and -80°C, with latter temperature allowing its use even years after its production.

Another advantage is the independence from blood donor availability. Especially during summer time, when the majority of donors is on vacation, nationwide short-cuts in blood supply reoccur.

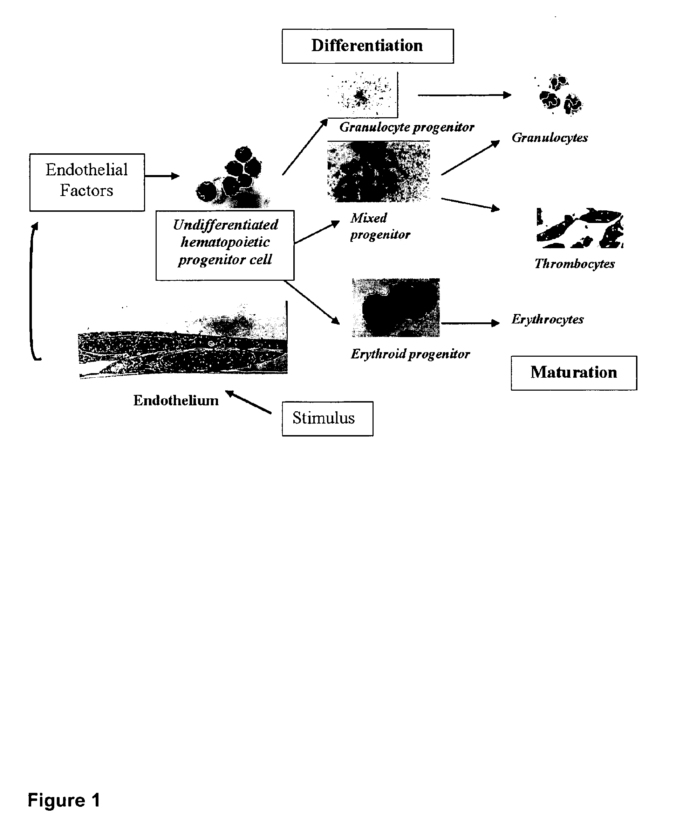

Figure 1 shows a schematic description of the approach of using the supernatant of stimulated EC and hematopoietic progenitor cells for the production of granulocytes, thrombocytes, and erythrocytes.

The invention is now further described by reference to the following examples which are intended to illustrate, not to limit the scope of the invention.

### Example 1

### Isolation and culture of HPC and EC

Human cord blood specimens were collected in heparin-coated syringes, 50 ml centrifuge tubes, or cord blood bags from full-term deliveries, after informed consent was obtained from the mothers. Mononuclear cell fractions were isolated by Ficol (Biochrom, Berlin, Germany), followed by two wash steps. Viable cells were determined by a hemocytometer after trypan blue exclusion, and CD34(+) HPC were immunomagnetically selected as previously described (Moldenhauer A. et al., Stem Cells 22 (2004) 283-291). Obtained CD34(+) HPC were cultured in stem cell medium consisting of basic stem cell medium (Iscove's modified Dulbecco's medium, IMDM, Biochrom) supplemented with 20% fetal bovine serum (FBS; Hyclone, South Logan, UT), 2 mM L-glutamine, 50 µg/ml gentamicin with or without 7.3 x 10⁻⁵ M mercaptoethanol.
Umbilical cord EC were obtained by flushing umbilical veins with 0.1% collagenase (Sigma-Aldrich, Steinheim, Germany) (Moldenhauer A. et al., Stem Cells 22 (2004) 144-157). Obtained EC were cultured in EC conditioning medium consisting of M199 (Biochrom, Berlin, Germany) supplemented with 16% FBS, 4% human serum from healthy volunteers, 2mM L-glutamine, 0.15 mg/ml endothelial growth factor supplement (Intracel, Rockville, MD), 0.015 mg/ml heparin, and 1% fungicide.

### Example 2

### Production of granulocytes

Confluent EC monolayers from passage 2-7 were stimulated with 1 to 10.000 U/ml interleukin-1 (Peprotech, Rocky Hill, MD) for 16 hours, before supernatants were collected, filtered through a 0.2 µm sterile filter, and diluted 1:2 with stem cell medium.
CD34(+) or CD133(+) HPC were cultured in the mix of interleukin-1 stimulated EC supernatant and stem cell medium, which was either used immediately or after short-term storage at 4°C or long-term storage at -80°C.
After 14 days in culture, cells were resuspended in stem cell medium including granulocyte-colony stimulating factor (G-CSF; 100ng/ml). Mature granulocytes were determined by phagoburst tests and determination of CD16 and CD66. From 1x10⁴ CD34(+) cells, 1x10⁷ mature granulocytes were achieved by further maturation in stem cell medium and addition of granulocyte-colony stimulating factor. Replacement of interleukin-1β with interleukin-3 resulted in similar expansion rates with a major development of eosinophilic granulocytes.

### Example 3

### Production of thrombocytes

For the production of thrombocytes, endothelial cells were stimulated for 16 hours with fibroblastic growth factor (or SDF-1 or thrombopoietin). Stimulated supernatants were harvested as mentioned above. CD34(+) or CD133(+) cells were cultured in the stimulated endothelial supernatant, and the presence of CD41 and CD42 positive cells was determined after one and two weeks. Expansion rates were 5- and 10-fold after one and two weeks, respectively. In a second approach, hematopoietic progenitor cells were expanded in either interleukin-1, interleukin-3 or interleukin 11-stimulated supernatant for 7 to 14 days. Then, expanded cells were further matured in stem cell medium plus thrombopoietin. Starting from 10⁵ cells, 10⁹ cells were achieved.

### Example 4

### Production of erythrocytes

For the production of erythrocytes, endothelial cells were stimulated for 16 hours with insulin like growth factor 1 (or IGF-2 or erythropoietin). Stimulated supernatants were harvested as mentioned above. CD34(+) or CD133(+) cells were cultured in the stimulated endothelial supernatant, and the presence of glycophorin A-positive cells was determined after one and two weeks. Expansion rates were 5- and 10-fold after one and two weeks, respectively. Maturation of erythrocytes was further enhanced by the addition of dexamethasone as the final maturation factor.
In a second approach, hematopoietic progenitor cells were expanded in interleukin-1 or interleukin-3 stimulated supernatant for 7 to 14 days. Then, expanded cells were further matured in basic stem cell medium plus erythropoietin. Starting from 10⁵ cells, more than 10¹⁰ cells were achieved.

### Example 5

### Yields of cellular blood components produced according to the present invention in comparison to standard yields achieved with ordinary blood donation or apheresis

**Table 1**

| **Yield by:** | **a single average donation / apheresis** | **the method according to the present invention** (starting from 10⁷ cells) | x-fold increase** |
|---|---|---|---|
| **Erythrocytes** | 2 x 10¹² | 3-5 x 10¹² | ∼ 300000-500000 |
| **Hemoglobin*** | 40-45 g | 80-90 g | n.a.*** |
| **Granulocytes** | 1 x 10¹⁰ | 1-2 x 10¹⁰ | ∼ 1000-2000 |
| **Thrombocytes** | 2-4 x 10¹¹ | 4-8 x 10¹¹ | ∼ 40000-80000 |

| | | | |
|---|---|---|---|
| * Usually, the number of erythrocytes collected in one blood donation is not a quality parameter. More important is the content of hemoglobin. ** This term refers to the increase of mature blood cells over the initial number of hematopoietic progenitor cells (10⁷). *** not applicable | | | |

## Claims

1. A method of producing a homologous cellular blood component, said method comprising:
collecting vein endothelial cells from the umbilical cord and hematopoietic progenitor cells from the umbilical cord blood of a single individual,
culturing said vein endothelial cells in the presence of a single stimulant, collecting the supernatant of said culture comprising said vein endothelial cells and said single stimulant,
culturing said hematopoietic progenitor cells in the presence of said supernatant, and
collecting said cellular blood component from said culture comprising said hematopoietic progenitor cells and said supernatant.

2. The method according to any of the foregoing claims, wherein said hematopoietic progenitor cells are CD34(+) or CD133(+) cells.

3. The method according to any of the foregoing claims, wherein said cellular blood component is selected from the group comprising granulocytes, thrombocytes, and erythrocytes.

4. The method according to claim 3, wherein said cellular blood component are granulocytes.

5. The method according to claim 4, wherein said single stimulant is interleukin-1 or interleukin-3.

6. The method according to claim 3, wherein said cellular blood component are thrombocytes.

7. The method according to claim 6, wherein said single stimulant is selected from the group comprising stroma-derived factor 1, thrombopoietin, fibroblastic growth factor 4, and interleukin-11.

8. The method according to claim 3, wherein said cellular blood component are erythrocytes.

9. The method according to claim 8, wherein said single stimulant is selected from the group comprising insulin-like growth factor 1, insulin-like growth factor 2, and erythropoietin.

10. The method according to any of the foregoing claims, wherein said vein endothelial cells are cultured in the presence of a single stimulant for 15 to 24 hours.

11. The method according to any of the foregoing claims, wherein said hematopoietic progenitor cells are cultured in the presence of said supernatant for ≤ 14 days.

12. The method according to any of the foregoing claims, wherein said hematopoietic progenitor cells are cultured in the presence of said supernatant and further matured after expansion by addition of a single maturation stimulant.

13. The method according to claim 12, wherein said single maturation stimulant is G-CSF for granulocytes, thrombopoietin for thrombocytes, and erythropoietin for erythrocytes.

14. The method according to claims 12 or 13, wherein, in relation to the initial number of said hematopoietic progenitor cells, the yield of said cellular blood component is:
a) in the case of granulocytes, more than 100-fold, preferably more than 500-fold, even more preferably more than 1000-fold,
b) in the case of thrombocytes, more than 1000-fold, preferably more than 5000-fold, even more preferably more than 10000-fold, and
c) in the case of erythrocytes, more than 10000-fold, preferably more than 50000-fold, even more preferably more than 100000-fold.

15. A homologous cellular blood component, selected from the group comprising granulocytes, thrombocytes and erythrocytes, and produced by the method according to any of claims 1 to 14.

16. Use of a homologous cellular blood component according to claim 15 for transfusion.
